# EUROPÄISCHE PATENTSCHRIFT

(11) **EP 0 678 286 B1**
(45) Veröffentlichungstag und Bekanntmachung des Hinweises auf die Patenterteilung: **17.12.1997**
(21) Anmeldenummer: 94106945.2
(22) Anmeldetag: 04.05.1994
(51) Int. Cl.: A61F 2/38

(54) **Kniegelenkprothese**
Knee joint prothesis
Prothèse d'articulation du genou

(30) Priorität: 22.03.1994 DE 4409787
(43) Veröffentlichungstag der Anmeldung: 25.10.1995
(73) Patentinhaber: ALPHANORM MEDIZINTECHNIK GmbH, D-66538 Neunkirchen (DE)
(72) Erfinder:
(74) Vertreter: Patentanwälte Hauck, Graalfs, Wehnert, Döring, Siemons

(56) Entgegenhaltungen:
- EP-A- 0 472 475
- DE-U- 9 212 879
- FR-A- 2 615 726
- GB-A- 2 223 950
- US-A- 4 224 697
- US-A- 5 147 405

## Beschreibung

Die Erfindung bezieht sich auf eine Kniegelenkprothese gemäß dem Oberbegriff von Anspruch 1.

Um eine relative Drehbewegung des Lagereinsatzes auf der Tibiakomponente zu gewährleisten, ist bekannt, daß der Lagereinsatz einen sich nach unten erstreckenden Drehzapfen aufweist, der in einen sich verjüngenden, hohlen Zapfen, der an der Unterseite der Tibiakomponente ausgebildet ist, eingreift. Ein solcher Drehzapfen, der auch getrennt von dem Lagereinsatz ausgebildet sein kann, ist beispielsweise in der EP-PS 0 018 364, der EP-A-0 568 756 oder in der EP-A-0 519 873 beschrieben.

Die DE-OS 3 642 576 beschreibt eine zweiteilige Kniegelenkprothese, wobei die Tibiakomponente an ihrer Oberseite einen Zwischenflächenabschnitt und zwei äußere, erhabene Flächenabschnitte aufweist. Die Trennwände zwischen diesen Flächenabschnitten sind gewölbt und die Unterseite des Lagereinsatzes ist komplementär ausgebildet. Die Zwischenräume zwischen den Rändern der Trennwände sind so bemessen, daß der Lagereinsatz zwischen ihnen durchgeführt und dann abgewinkelt in seine Einbaulage bewegt werden kann, wobei die Trennwände Nut- und Federverbindungen aufweisen können.

Aus der EP-A-0 472 475 ist eine Kniegelenkprothese mit den Merkmalen des Oberbegriffes des Patentanspruches 1 bekannt. Der bei dieser Prothese mit dem Tibiaplateau zusammenwirkende Lagereinsatz weist eine Ausnehmung auf, in die ein entsprechender Verbindungsansatz eingreift. An eine Fixierung des Lagereinsatzes in Axialrichtung relativ zum Tibiaplateau ist jedoch nicht gedacht.

Aus der DE-U-9 212 879 ist es bei einer Kniegelenkendoprothese bekannt, den Lagereinsatz an der Tibiakomponente zu arretieren. Hierzu findet als weiteres Bauteil eine auf einer Erhebung der Tibiakomponente aufzusetzende Kappe Verwendung, die mit Hilfe einer Schraube zu sichern ist. Die Kappe besitzt einen seitlichen Kragen, der sich über einen Bund des Lagereinsatzes erstreckt und somit eine Bewegung desselben in Richtung auf die Femurkomponente verhindert.

Der Erfindung liegt die Aufgabe zugrunde, eine Kniegelenkprothese der angegebenen Art zu schaffen, bei der eine verschleiß- und reibungsarme Rotation zwischen dem Lagereinsatz und der Tibiakomponente und eine Arretierung des Lagereinsatzes in Richtung auf die Femurkomponente mit einfachen Mitteln gewährleistet ist.

Diese Aufgabe wird erfindungsgemäß bei einer Kniegelenkprothese der angegebenen Art durch die kennzeichnenden Merkmale des Patentanspruches 1 gelöst.

Die Kniegelenkprothese nach der Erfindung weist eine Femurkomponente, eine Tibiakomponente mit einer Plattform und einen zwischen Femur- und Tibiakomponente angeordneten Lagereinsatz auf. Von der Plattform der Tibiakomponente erstreckt sich in Richtung des Lagereinsatzes ein Verbindungsansatz. Der Lagereinsatz weist an seiner Unterseite eine Ausnehmung auf, um den Lagereinsatz auf dem Verbindungsansatz der Tibiakomponente anzuordnen.

Der Verbindungsansatz ist in Form eines sich nach oben erstreckenden Flansches einstückig mit der Tibiakomponente ausgebildet. Der Flansch weist an seiner Außenseite eine Nut auf, in der an der Innenwand der Ausnehmung ausgebildete Rippen laufen können, so daß der Lagereinsatz auf der Tibiakomponente arretiert ist.

Zur Begrenzung der relativen Drehbewegung des Lagereinsatzes zu der Tibiakomponente kann der Flansch einen vorspringenden Anschlag aufweisen, wobei die Ausnehmung mindestens eine Erweiterung entlang eines Teils ihres Innenumfangs aufweist. Der Anschlag kann in dieser Erweiterung laufen, bis er an eine Endfläche der Erweiterung anstößt. Damit wird die Rotationsmöglichkeit begrenzt.

Ein Vorteil der Kniegelenkprothese nach der Erfindung ist ihr modulartiger Aufbau sowie die Möglichkeit ihres stufenweisen Ausbaues bis hin zu einer achsgeführten Kniegelenkprothese.

So können der Tibiaschaft und der Femurschaft zur Verankerung in der Tibia bzw. in dem Femur in Anpassung an die jeweiligen anatomischen Gegebenheiten eine unterschiedliche Form und Erstreckung aufweisen. Ebenso ist der Lagereinsatz frei auswählbar, so daß dessen Höhe und Form in Abhängigkeit von der Größe des Gelenkspaltes bestimmt wird.

In einer ersten Stufe kann der Lagereinsatz auf der Tibiakomponente angeordnet, vorzugsweise aufgeclipst werden, so daß eine Drehung um die Erhebung der Tibiakomponente möglich ist. Eine freie Beweglichkeit ist somit unterdrückt, da nur noch freie Rotation möglich ist. Bei nachlassender Spannung des Bandapparates oder insuffizientem hinteren Kreuzband ist dieser Schritt notwendig.

Zur besseren Führung der Femurkomponente kann eine zweite Ausbaustufe der Kniegelenkprothese nach der Erfindung eine Nase aufweisen, die sich von dem Lagereinsatz nach oben zwischen die gewölbten Flächen der Femurkomponente erstreckt. Zusätzlich kann zur Verstärkung der Anordnung noch beispielsweise ein Zapfen vorgesehen sein, der sich ggf. von dem Tibiaschaft durch den Zapfen der Tibiakomponente bis in die Nase des Lagereinsatzes erstreckt. Diese Stufe ist notwendig, wenn auch die Seitenbandstabilität nicht mehr ausreichend gegeben ist.

Eine dritte Stufe, die einer achsgeführten Kniegelenkprothese entspricht, wird dadurch erzielt, daß sich der Zapfen oder ein Achsmechanismus weiter durch die Nase hindurch in die Femurkomponente erstreckt und dort be-festigt wird.

Insbesondere ermöglicht die Kniegelenkprothese nach der Erfindung mit einfachen Mitteln eine verschleiß- und reibungsarme Rotation des vorzugsweise aus Polyethylen bestehenden Lagereinsatzes relativ zu der vorzugsweise aus einer CrCo-Legierung bestehenden Tibiakomponente bei gleichzeitiger Arretierung des Lagereinsatzes in Richtung der Femurkomponente. Dabei kann die Rotation durch die oben beschriebenen Mittel begrenzt werden.

Ausführungsbeispiele der Erfindung werden im folgenden anhand von Zeichnungen näher erläutert.
- Figur 1: zeigt im Querschnitt eine Tibiakomponente gemäß einer ersten Ausführungsform einer Kniegelenkprothese nach der Erfindung.
- Figur 2: zeigt in Draufsicht die Tibiakomponente nach Figur 1.
- Figur 3: zeigt in Draufsicht einen Lagereinsatz gemäß einer weiteren Ausführungsform einer Kniegelenkprothese nach der Erfindung.
- Figur 4: zeigt im Schnitt den Lagereinsatz entlang der Linie A-A gemäß Figur 3.
- Figur 5: zeigt den Ausschnitt X nach Figur 3 in vergrößerter Darstellung.
- Figur 6: zeigt im Schnitt eine Ausführungsform einer Kniegelenkendoprothese nach der Erfindung mit der Tibiakomponente gemäß Figur 1 und dem Lagereinsatz gemäß Figur 3, wobei die Femurkomponente nicht dargestellt ist.
- Figur 7: zeigt eine Fixierungsschraube der Ausführungsform nach Figur 6.
- Figur 8: zeigt im Schnitt eine weitere Ausführungsform der Kniegelenkprothese nach der Erfindung, wobei die Femurkomponente nicht dargestellt ist.
- Figur 9: zeigt einen Armierungszapfen der Ausführungsform nach Figur 8.

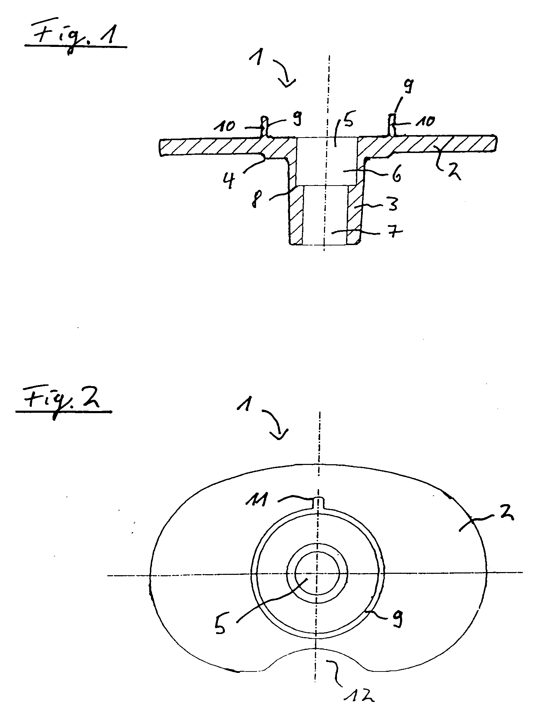

Die Figur 1 zeigt eine Tibiakomponente 1 mit einer Plattform 2 und mit einem an der Unterseite der Tibiakomponente angeordneten Zapfen 3. Der Zapfen 3 ist im wesentlichen zylindrisch ausgebildet und liegt einteilig mit der Plattform vor. An der Unterseite der Plattform 2 ist beabstandet von dem Zapfen 3 ein Vorsprung 4 ausgebildet.

Die Tibiakomponente weist ferner eine sich durch den Zapfen 3 erstreckende Bohrung 5 auf, deren oberer Abschnitt 6 einen größeren Durchmesser aufweist als deren unterer Abschnitt 7, wobei diese beiden Abschnitte 6, 7 durch einen Absatz 8 voneinander getrennt sind.

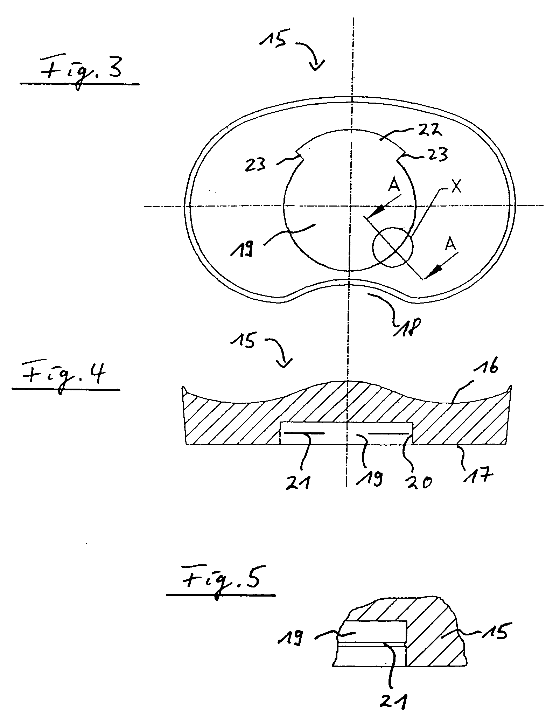

Von der Oberseite der Plattform 2 erstreckt sich ein umlaufender Flansch 9, dessen Wandung gegenüberliegend zu dem Vorsprung 4 ausgebildet ist. An einem mittleren Abschnitt weist der Flansch 9 an seiner Außenfläche eine umlaufende Nut 10 auf. Gemäß Figur 2 hat der Flansch 9 ferner einen zu der Vorderseite der Tibiakomponente vorspringenden Anschlag 11. In Draufsicht weist die aus einer CrCo-Legierung bestehende Tibiakomponente 1 einen im wesent-lichen ovalen Umriß mit einem hinteren Ausschnitt 12 auf.

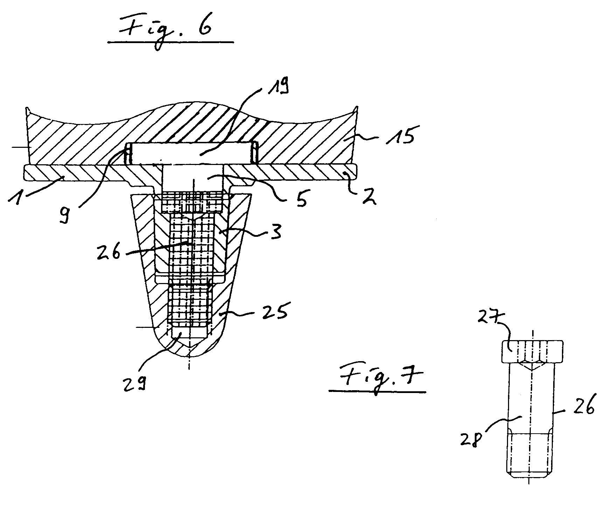

Die Figur 3 zeigt einen im wesentlichen ovalen Lagereinsatz 15 aus Polyethylen. An der Oberseite des Lagereinsatzes sind zwei Gleitlager 16 zur Lagerung der Kondylen einer nicht gezeigten Femurkomponente ausgebildet. Die Femurkomponente kann die übliche Form mit zwei beabstandeten, zu den Gleitlagern komplementären, gewölbten Flächen aufweisen.

Eine untere Lagerfläche 17 ist in Anpassung an die Oberseite der Plattform 2 der Tibiakomponente 1 eben ausgeführt. Ebenfalls in Anpassung an den hinteren Ausschnitt 12 der Tibiakomponente 1 hat der Lagereinsatz 15 einen hinteren Ausschnitt 18.

Eine an der Unterseite des Lagereinsatzes vorgesehene Ausnehmung 19 weist einen Durchmesser sowie eine Tiefe auf, deren Dimensionen im wesentlichen denen des Durchmessers des Flansches 9 sowie dessen Höhenerstreckung entsprechen. Die Dimensionen sind dabei so gewählt, daß der Lagereinsatz 15 auf die Tibiakomponente 1 aufgeclipst werden kann.

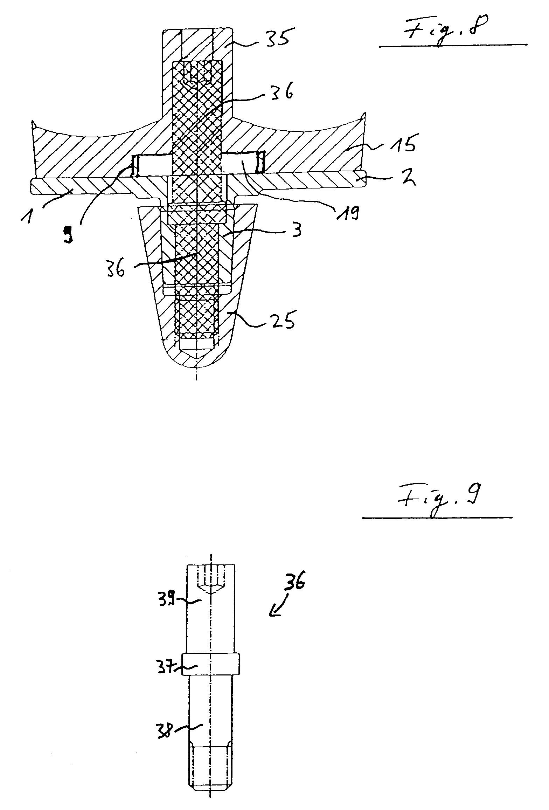

An den inneren Wänden 20 der Ausnehmung 19 sind insgesamt vier Rippen 21 vorgesehen, von denen zwei schematisch in der Figur 4 dargestellt sind. Die Figur 5 zeigte einen Ausschnitt mit einer entsprechend vergrößert gezeigten Rippe 21.

In Abweichung von der Form des Flansches 9 der Tibiakomponente 1 hat die Ausnehmung 19 an der Vorderseite des Lagereinsatzes eine Erweiterung 22, die sich etwa entlang eines Viertels des Umfanges der Ausnehmung erstreckt und die durch Endflächen 23 begrenzt ist.

Der Lagereinsatz 15 kann gemäß Figur 6 auf die Tibiakomponente 1 aufgeclipst werden, wobei die vier Rippen 21 des Lagereinsatzes in der Nut 10 des Flansches 9 der Tibiakomponente laufen und wodurch der Lagereinsatz auf der Tibiakomponente arretiert ist. Die untere Lagerfläche 17 des Lagereinsatzes steht dabei in Gleitkontakt mit der Oberseite der Plattform 2 der Tibiakomponente. Die Montage des Lagereinsatzes auf der Tibiakomponente erfolgt mit einem speziellen Einschläger.

Die mögliche Drehbewegung des Lagereinsatzes relativ zu der Tibiakomponente wird durch den Anschlag 11 des Flansches 9 begrenzt. Der Anschlag 11 kann in der Erweiterung 22 laufen und so eine relative Drehbewegung gewährleisten, bis er an eine der Endflächen 23 der Erweiterung anstößt, wobei diese Position die maximale Drehposition darstellt.

Auf den Zapfen 3 der Tibiakomponente 1 ist ein ausgewählter Tibiaschaft 25 aufgesteckt, wobei die Sicherung des Schaftes 25 an dem Zapfen 3 über eine, in der Figur 7 getrennt dargestellte Fixierungsschraube 26 erfolgt. Der obere, verbreiterte Abschnitt 27 der Fixierungsschraube 26 ist in dem oberen, verbreiterten Abschnitt 6 der Bohrung 5 der Tibiakomponente 1 angeordnet, während sich der untere Abschnitt 28 der Schraube 26 durch den unteren Abschnitt 7 der Bohrung 5 und weiter in eine Bohrung 29 des Tibiaschaftes erstreckt und dort fixiert wird.

Die in der Figur 8 gezeigte Ausführungsform einer Kniegelenkprothese weist im wesentlichen die gleichen Bauteile wie die Ausführungsform gemäß Figur 6 auf. Gleiche Bauteile sind mit gleichen Bezugsziffern gekennzeichnet. Es werden hier nur die wesentlichen Unterschiede erläutert, ansonsten wird auf die obige Beschreibung verwiesen.

Der Lagereinsatz 15 weist eine Nase 35 auf, die sich zwischen den zwei Gleitlagern 16 zur Lagerung der Kondylen einer nicht gezeigten Femurkomponente nach oben in Richtung der Femurkomponente erstreckt. Die Nase kann dabei die Femurkomponente führen. Ein Arretierungszapfen 36, der in der Figur 9 einzeln dargestellt ist, erstreckt sich von dem Tibiaschaft 25, in dem er befestigt ist, durch den Zapfen 3 der Tibiakomponente 1 und von dort in eine in der Nase 35 ausgebildeten Bohrung. Ein mittlerer Abschnitt 37 und ein unterer Abschnitt 38 entsprechen im wesentlichen den Abschnitten 27 und 28 der Fixierungsschraube 26 gemäß Figur 7.

Die Länge eines oberen Abschnitts 39 des Armierungszapfens 36 wird in Abhängigkeit von der Höhe des Lagereinsatzes 15 gewählt, da dieser, wie bei der Ausführungsform gemäß Figur 6, in Anpassung an die anatomischen Verhältnisse frei ausgewählt werden kann.

Der Armierungszapfen 36 kann sich ferner durch die Nase 35 in die Femurkomponente erstrecken und dort befestigt werden (nicht gezeigt), so daß eine achsgeführte Kniegelenkendoprothese entsteht.

## Patentansprüche

1. Kniegelenkprothese mit einer Femurkomponente, einer Tibiakomponente (1) mit einer Plattform (2) und einem zwischen der Femur- und Tibiakomponente (1) angeordneten Lagereinsatz (15), wobei sich von der Plattform (2) der Tibiakomponente (1) ein hiermit einstückig ausgebildeter ringförmiger Flansch (9) als Verbindungsansatz in Richtung des Lagereinsatzes (15) erstreckt und der Lagereinsatz (15) an seiner Unterseite eine Ausnehmung (19) zur drehbaren Lagerung des Lagereinsatzes (15) auf dem Flansch (9) der Tibiakomponente (1) aufweist, dadurch gekennzeichnet, daß der einstückige Flansch (9) an seiner Außenseite mindestens eine Nut (10) aufweist, die sich in Umfangsrichtung zumindest entlang eines Teiles des Außenumfanges des Flansches (9) erstreckt, und daß an der Innenwand (20) der Ausnehmung (19) mindestens eine Rippe (21) ausgebildet ist, die sich zumindest entlang eines Teiles des Innenumfanges der Ausnehmung (19) erstreckt und in die Nut (10) eingreift.

2. Kniegelenkprothese nach Anpruch 1, dadurch gekennzeichnet, daß der Flansch (9) einen in radialer Richtung vorspringenden Anschlag (11) aufweist.

3. Kniegelenkprothese nach einem der vorangehenden Ansprüche, dadurch gekennzeichnet, daß vier beabstandete Rippen (21) an der Innenwand der Ausnehmung (19) ausgebildet sind, die sich jeweils entlang eines Teiles des Innenumfanges der Ausnehmung (19) erstrecken.

4. Kniegelenkprothese nach einem der vorangehenden Ansprüche, dadurch gekennzeichnet, daß entlang eines Teiles des Innenumfanges der Ausnehmung (19) mindestens eine radiale Erweiterung (22) ausgebildet ist.

5. Kniegelenkprothese nach einem der vorangehenden Ansprüche, dadurch gekennzeichnet, daß die Tibiakomponente (1) einen sich in Richtung Tibia erstreckenden Zapfen (3) aufweist, der eine Bohrung (5) hat.

6. Kniegelenkprothese nach Anspruch 5, dadurch gekennzeichnet, daß eine in die Bohrung (5) einführbare Fixierungsschraube (26) zur Verbindung der Tibiakomponente (1) mit einem ausgewählten Tibiaschaft (25) vorgesehen ist.

7. Kniegelenkprothese nach einem der vorangehenden Ansprüche, dadurch gekennzeichnet, daß der Lagereinsatz (15) an seiner Oberseite eine sich in Richtung Femur erstreckende Nase (35) aufweist.

8. Kniegelenkprothese nach Anspruch 7, dadurch gekennzeichnet, daß ein Armierungszapfen (36) vorgesehen ist, der sich in die Nase (35) erstreckt.

9. Kniegelenkprothese nach Anspruch 7 oder 8, dadurch gekennzeichnet, daß sich der Armierungszapfen (36) von dem Tibiaschaft (25) durch den Zapfen (3) der Tibiakomponente (1) in die Nase (35) erstreckt.

10. Kniegelenkprothese nach einem der vorangehenden Ansprüche, dadurch gekennzeichnet, daß ein Zapfen (36) mit Achse die Tibiakomponente (1) mit der Femurkomponente verbindet.

## Claims

1. A knee joint prothesis comprising a femur component, a tibia component (1) having a platform (2), and a bearing insert (15) disposed between the femur component and the tibia component (1), wherein an annular flange (9) integrally formed with the platform (2) of the tibia component (1) extends from the platform (2) of the tibia component (1) as connection stud towards the bearing insert (15), and the bearing insert (15) has a recess (19) on its lower side for rotatably supporting the bearing insert (15) on the flange (9) of the tibia component (1), characterized in that the integral flange (9) has at least one groove (10) on its exterior side, said groove extending in circumferencial direction at least along a part of the outer circumference of the flange (9), and it that at least one rib (21) is formed on the inner wall (20) of the recess (19) and extends at least along a part of the inner circumference of the recess (19) and engages into the groove (10).

2. The knee joint prothesis according to claim 1, characterized in that the flange (9) has a stop (11) projecting in a radial direction.

3. The knee joint prothesis according to one of the preceding claims, characterized in that four spaced ribs (21) are formed on the inner wall of the recess (19) and extend along a part of the inner circumference of the recess (19), respectively.

4. The knee joint prothesis according to one of the preceding claims, characterized in that at least one radial enlargement (22) is formed along a part of the inner circumference of the recess (19).

5. The knee joint prothesis according to one of the preceding claims, characterized in that the tibia component (1) has a peg (3) extending towards the tibia and having a bore (5).

6. The knee joint prothesis according to claim 5, characterized in that a fixation screw (26) introduceable into the bore (5) is provided for the connection of the tibia component (1) with a selected tibia shaft (25).

7. The knee joint prothesis according to one of the preceding claims, characterized in that the bearing insert (15) has a lug (35) on its upper side, said lug extending towards the femur.

8. The knee joint prothesis according to claim 7, characterized in that an armouring peg (36) is provided which extends into the lug (35).

9. The knee joint prothesis according to claim 7 or 8, characterized in that the armouring peg (36) extends from the tibia shaft (25) through the peg (3) of the tibia component (1) into the lug (35).

10. The knee joint prothesis according to one of the preceding claims, characterized in that a peg (36) with axis connects the tibia component (1) with the femur component.

## Revendications

1. Prothèse de l'articulation du genou, comportant un composant de fémur, un composant de tibia (1) doté d'une plate-forme (2) et d'une garniture de montage (15) disposée entre le composant de fémur et le composant de tibia (1), une bride (9) de forme annulaire, servant d'appendice de liaison en direction de la garniture de montage (15), partant de la plate-forme (2) du composant du tibia (1) et réalisée d'un seul tenant avec cette dernière, et la garniture de montage (15) présentant sur sa face inférieure un évidement (19) servant à monter la garniture de montage (15) à rotation sur la bride (9) du composant de tibia (1), caractérisée en ce que la bride (9) d'une seule pièce présente sur sa face externe au moins une rainure (10) qui s'étend dans la direction périphérique au moins sur une partie de la périphérie externe de la bride (9), et en ce que sur la paroi interne (20) de l'évidement (19) est formée au moins une nervure (21) qui s'étend au moins sur une partie de la périphérie interne de l'évidement (19) et qui s'engage dans la rainure (10).

2. Prothèse d'articulation du genou selon la revendication 1, caractérisée en ce que la bride (9) présente une butée (11) en saillie dans la direction radiale.

3. Prothèse d'articulation du genou selon l'une des revendications précédentes, caractérisée en ce que quatre nervures (21) situées à distance l'une de l'autre sont formées sur la paroi interne de l'évidement (19) et s'étendent chacune sur une partie de la périphérie interne de l'évidement (19).

4. Prothèse d'articulation du genou selon l'une des revendications précédentes, caractérisée en ce qu'au moins un évasement radial (22) est formé sur une partie de la périphérie interne de l'évidement (19).

5. Prothèse d'articulation du genou selon l'une des revendications précédentes, caractérisée en ce que le composant de tibia (1) présente un tourillon (3) qui s'étend dans la direction du tibia et présente un alésage (5).

6. Prothèse d'articulation du genou selon la revendication 5, caractérisée en ce qu'il est prévu une vis de fixation (26) qui peut être insérée dans l'alésage (5) pour relier le composant de tibia (1) à une tige de tibia (25) sélectionnée.

7. Prothèse d'articulation du genou selon l'une des revendications précédentes, caractérisée en ce que la garniture de montage (15) présente sur sa face supérieure un bec (35) qui s'étend en direction du fémur.

8. Prothèse d'articulation du genou selon la revendication 7, caractérisée en ce qu'il est prévu un tourillon de renfort (36) qui s'étend dans le bec (35).

9. Prothèse d'articulation du genou selon les revendications 7 ou 8, caractérisée en ce que le tourillon de renfort (36) part de la tige de tibia (25) et traverse le tourillon (3) pour s'étendre jusque dans le bec (35).

10. Prothèse d'articulation du genou selon l'une des revendications précédentes, caractérisée en ce qu'un tourillon (36) avec axe relie le composant de tibia (1) au composant de fémur.
